# EUROPÄISCHE PATENTANMELDUNG

(11) **EP 1 010 981 A1**
(43) Veröffentlichungstag der Anmeldung: **21.06.2000**
(21) Anmeldenummer: 98124131.8
(22) Anmeldetag: 18.12.1998
(51) Int. Cl.: G01N 33/569, G01N 33/532, A61K 39/12, A61K 39/235, C12N 7/02

(54) **Konjugate zum Nachweis von Viren und deren Verwendung**

(71) Anmelder: Diagor GmbH, 56856 Zell/Mosel (DE)
(72) Erfinder: Opitz, Georg, Dr., 69469 Weinheim (DE); Höcker, Hartwig, Prof.Dr., 52062 Aachen (DE)
(74) Vertreter: Schmidt-Evers, Jürgen, Dipl.-Ing.

(57) **Zusammenfassung**

Die Erfindung betrifft ein Konjugat bestehend aus einem toxikologisch unbedenklichen hochmolekularen Träger und einer spezifisch Virus-bindenden Substanz, dessen Verwendung und einem Verfahren zum Anreichern und anschließendem oder gleichzeitigen Nachweis von Viren. Die Erfindung betrifft im besonderen ein Verfahren zur Anreicherung und Diagnose von Influenza A/B Viren.

## Beschreibung

Die Erfindung betrifft ein Konjugat bestehend aus einem toxikologisch unbedenklichen hochmolekularen Träger und einer spezifisch Virus-bindenden Substanz, dessen Verwendung und einem Verfahren zum Anreichern und anschließendem oder gleichzeitigen Nachweis von Viren. Die Erfindung betrifft im besonderen ein Verfahren zur Anreicherung und Diagnose von Influenza A/B Viren.

### Beschreibung

Viren im Nase-, Mund- und Rachenraum führen häufig zu Erkrankungen und sind nicht einfach nachzuweisen. Dies gilt insbesondere für Infuenza A/B Viren.

Influenza A/B Viren sind verantwortlich für die Grippe, an der jährlich mehrere 100 Millionen Menschen weltweit erkranken. Die Influenza-A/B-Virus-bedingte Grippe läßt sich nur schwer von anderen akuten respiratorischen Infekten unterscheiden. Möglich ist dies nur mit Influenza-A/B-Virus-spezifischen Nachweisverfahren. Ein besonderes Problem besteht ,indes darin, aus den befallenen Bereichen, d. h. Rachen und Nase, Material zu bekommen, das für einen Nachweis ausreichende Mengen an Influenza A/B Virus enthält. Normalerweise werden entsprechende Proben in Form von Abstrichen, Waschungen oder Aspiraten gewonnen. Diese Entnahmen von Proben sind nur von geschultem Personal durchführbar. Wünschenswert wäre ein Verfahren zur Gewinnung von Influenza-A/B-Virus-haltigem Probenmaterial und deren Nachweis durch den Patienten selbst.

Die vorliegende Erfindung beschreibt eine einfache Möglichkeit Viren in ausreichenden Mengen zu isolieren und nachzuweisen. Als Viren im Sinne der Erfindung gelten insbesondere Viren des Halses, Mundes, Rachen und der Nase. Dies sind Influenza A und Influenza B Viren, Respiratorischer Syncytialvirus, Parainfluenza Viren, Rhinoviren und Adenoviren. Bevorzugt nachzuweisende Viren sind Influenza A oder B Viren.

Die Viren werden durch Konjugate bestehend aus einem toxikologisch unbedenklichem hochmolekularen Träger und einer Viren bindenden Substanz angereichert. Die hochmolekularen Träger sind insbesondere ein toxikologisch unbedenkliches Polymer mit kopplungsfähigen Gruppen, ausgewählt aus der Gruppe bestehend aus Naturkautschuk, Silikonkautschuk und Polyolefine und die Derivate dieser Polymere. Die Konjugate werden in Form von Kaugummi oder Lutscher zur Bindung und Anreicherung von Virus aus dem Speichel von Patienten verwendet. Das an das Polymerkonjugat gebundene Virus wird direkt über seine enzymatische Aktivität oder nach Desorption mit einem in vitro Virusschnelltest bestimmt. Es sind verschiedene in vitro Schnelltests allgemein zugänglich.

Im folgenden wird die Erfindung am Beispiel des Influenza A/B Virus erläutert. Der Fachmann kann die erfindungsgemäßen Ausführungsformen an andere Viren anpassen.

Influenza A/B Virus wird von infizierten Zellen des Atmungstraktes freigesetzt. Ein Teil des Virus verbleibt auf der Oberfläche der Zellen, der andere Teil gelangt in den Speichel bzw. das Nasensekret, was letztlich dafür verantwortlich ist, daß über die Bildung kleiner Influenza-A/B-Virus-haltiger Tröpfchen beim Ausatmen, vor allem aber durch Husten oder Niesen, das Virus verbreitet wird ( Tröpfcheninfektion ). Mit Hilfe spezifischer Influenza-A/B-Virus-bindender Moleküle ist es möglich, im Rachen und Mund befindliches Influenza A/B Virus zu binden, anzureichern und zu diagnostizieren. Dazu werden an toxikologisch unbedenkliche, wasserunlösliche Polymere die spezifisch Influenza-A/B-Virus-bindenden Moleküle chemisch gekoppelt. Bevorzugt sind kovalente Konjugate. Als dafür geeignet erwies sich u. a. Neuraminsäure und deren Derivate, insbesondere (-2-O-methyl-5-N-thioacetylneuraminsäure, sowie Neuraminidase Inhibitoren vom Typ Zanamivir (4-guanidinoNeu5Acen) oder GS 4071 ((3R,4R,5S)-4-acetamido-5-amino-3-(1-ethylpropoxy)-1-cyclohexane-carboxylsäure) oder spezifische Antikörper gegen Influenza A/B Virus, insbesondere monoklonale Antikörper.

Als Bindungspartner sind Polymere geeignet, die einerseits in jeder Beziehung ungiftig sind und andererseits kautschukelastische Eigenschaften besitzen. Ein naheliegendes Beispiel ist der in Kaugummis benutzte Naturkautschuk. Geeigneter ist Silikonkautschuk, der besser definiert und durch Vernetzung hinsichtlich seiner Eigenschaften besser einstellbar ist. Darüber hinaus sind auch Polyolefine in Form von Weichschaum einsetzbar. Sie weisen eine besonders große Oberfläche auf. Die erwähnten Polymere sollen als Beispiele gelten; grundsätzlich lassen sich durch entsprechende Modifizierung nahezu alle Polymere so einstellen, daß sie durch kautschukelastische Eigenschaften gekennzeichnet sind und diese auch während des Gebrauchs beibehalten.
Bei der Probenentnahme in der Nase müssen hochmolekulare Trägermaterialien mit hoher Oberfläche und/oder hoher Belegung der Virus-bindenden Substanz verwendet werden. Das Konjugat wird dann zum Beispiel in Form eines Tupfers oder dünnen elastischen Stabes verwendet.

Wird das Konjugat im Mundraum verwendet, so ist biß- und kaustabiles hochmolekulares Trägermaterial bevorzugt. Das Influenza A/B Virus spezifisch bindende Molekül ist auf der Oberfläche der oben genannten kautschukelastischen Materialien zu immobilisieren. Hierzu ist zunächst die Schaffung von funktionellen Gruppen auf der Oberfläche des Polymermaterials erforderlich. Dies geschieht z.B. durch Behandlung mit einem Plasma zur Erzeugung von Polymer-gebundenen Radikalen, die in der Lage sind, die radikalische Polymerisation geeigneter Monomerer, z.B. von Acrylsäure zu initiieren. Auf diese Weise werden auf der Oberfläche funktionelle Gruppen, im vorliegenden Fall Carboxygruppen erzeugt, die nach Methoden der Peptidchemie mit Aminogruppen zu Amidbindungen umgesetzt werden können.

Als Molekül, das sich zur Anbindung an die Carboxylgruppen des modifizierten Kautschuks eignet, ist z.B. (-2-O-methyl-5-N-thioacetylneuraminsäure anzusehen, das mit Aminothioethanol zu dem entsprechenden Amin umgesetzt werden kann. Alternativ lassen sich die Carboxylgruppen der auf der Oberfläche gepfropften

Polyacrylsäure unter Anwendung der Cabodiimidmethode (EDC) mit Glycol-2,2-diaminodiethylether als Spacer umsetzen, wobei eine Aminogruppe für weitere Reaktionen frei bleibt, z.B. mit dem N-Hydroxysuccinimid-Aktivester, der 3-Acetylmercatopropionsäure. Nach Deacetylierung mit Hydroxylamin kann die Mercaptogruppe im Sinne einer Michael-Addition an die Doppelbindung der Maleinimidgruppe, die an den Wirkstoff gebunden ist, addiert werden.

Von wesentlicher Bedeutung ist die Dichte der auf der Oberfläche des Polymers befindlichen Influenza-A/B-Virus-bindenden Moleküle, z.B. eines Neuraminsäurederivats. Diese läßt sich einstellen, indem ein entsprechendes Mischungsverhältnis von Acrylsäure mit Acrylamid eingesetzt wird. Mit zunehmenden Acrylamidanteil kann die Konzentration der Carboxygruppen auf dem Polymer und damit die Dichte der Neuraminsäuregruppen auf der Oberfläche des kautschukelastischen Polymers reduziert werden.
Zur Bindung und Anreicherung von Influenza A/B Virus im Mundraum erweist sich das Polymerkonjugat in Form von Kaugummi bzw Lutscher als am besten geeignet. Der Lutscher bzw. das Kaugummi ist so gestaltet, daß die Virus-bindenden Moleküle sich auf ihrer Oberfläche befinden. Die Form eines Lutscher bietet sich an, da damit auch bei Kindern - einer wichtigen Patientengruppe - eine Probengewinnung problemlos möglich ist. Durch entsprechende Formgebung läßt sich ausschließen, daß der Lutscher verschluckt werden kann. Falls erforderlich, ließe sich sogar zur besseren Akzeptanz bei Kindern das kautschukelastische Polymer des Lutschers mit Geschmackstoffen versehen. Das Material des Konjugats sollte beiß/kaufähig sein, da über die gesteigerte Speichelbildung beim Kauen mehr Viren vom infizierten Gewebe des Rachenraums freigesetzt werden. Die so im Speichel erhöhte Virusdichte führt zu einer verstärkten Anreicherung von Viren auf dem Konjugat. Das Influenza-A/B-Virus-bindende Konjugat wird biszu 30 Minuten dem Speichel ausgesetzt, bevorzugt bis zu 15 Minuten, insbesondere etwa 3 bis 7 Minuten.

Die Bindung des Virus an das Polymerkonjugat ist abhängig von der Inkubationszeit und Viruskonzentration. Eine 10 minütige Inkubationzeit erweist sich als ausreichend. Nachdem das Virus vom Polymerkonjugat durch geeignete Detergentien (z.B. Triton X-100) gelöst und aufgeschlossen wurde, läßt sich das mit Polymerkonjugat gewonnene Influenza A/B Virus mit einem der üblichen diagnostischen Influenza A/B in vitro Schnelltests nachweisen.

Der Nachweis von Influenza A/B Virus kann immunologisch erfolgen, bevorzugt mittels Influenza-A/B-spezifischen, mit Enzym gekoppelten Antikörpern.

Der Nachweis von Influenza A/B Virus kann außerdem enzymatisch erfolgen, bevorzugt über die Neuraminidaseaktivität des Virus in einem chromogenen oder fluorogenen Test.

Das auf dem Polymerkonjugat gebundene Influenza A/B Virus kann auch direkt über die enzymatische Aktivität der für Influenza A/B Virus charakteristischen Neuraminidase nachgewiesen werden. Dazu wird ein chromogenes Substrat der Neuraminidase mit dem Influenza A/B Virus haltigen Polymerkonjugat inkubiert. Eine mit der enzymatischen Spaltung des chromogenen Substrates einher gehende Farbänderung zeigt die Anwesenheit von Influenza A/B Virus an.

### Beispiel:

Eine Polydimethylsiloxan-Folie wird unter Standardbedingungen 15 sec im Argonplasma behandelt und anschließend der Luft ausgesetzt. Daraufhin wird die Folie in die wäßrige Lösung von Acrylsäure überführt und die Propfkopolymerisation thermisch oder photochemisch initiiert. Wenn sich die wäßrige Lösung zu trüben beginnt, wird die Folie der Lösung entnommen und mit Wasser gespült. Anschließend wird mit wäßriger Lösung von EDC umgesetzt und schließlich mit Glycol-2,2-diaminoethylether umgesetzt. Nach Spülen wird mit einem Überschuß des N-Hydroxysuccinimidaktivesters der 3- Acetylmercaptopropionsäure umgesetzt.

Anschließend wird gewaschen und mit 25 mmolarer Hydroxylaminlösung deacetyliert. Schließlich wird das biochemisch aktive Agens mit einer N-terminalen Maleinsäureimidgruppe hinzugefügt und im Sinne einer Michael-Addition gebunden.

Ein Quadratzentimeter der so hergestellten Polymerkonjugatfolie wird in Gefäße mit 5ml Nährlösung getaucht, die verschiedene Konzentrationen von Influenza A/B Virus enthalten. Die Folie wird 10 Minuten lang inkubiert. Mit einem Influenzaschnelltest läßt sich das in der Nährlösung und an dem Polymerkonjugat gebundene Virus nachweisen. Über die Polymerkonjugatfolie läßt sich das Influenza A/B Virus in einer Konzentration noch nachweisen, die 10-fach niedriger ist als die, die sich in der ursprünglichen Nährlösung mit dem Test nachweisen läßt. Mittels der Polymerkonjugatfolie ließ sich damit eine zehnfache Anreicherung des Virus erreichen.

## Patentansprüche

1. Konjugat aus einem toxikologisch unbedenklichen hochmolekularen Träger und einer Viren bindenden Substanz.

2. Konjugat nach Anspruch 1, wobei der hochmolekulare Träger ein toxikologisch unbedenkliches Polymer mit kopplungsfähigen Gruppen, ausgewählt aus der Gruppe bestehend aus Naturkautschuk, Silikonkautschuk und Polyolefine und deren Derivaten ist.

3. Konjugat nach einem der Ansprüche 1 oder 2, wobei der Virus ausgewählt ist aus der Gruppe bestehend aus Influenza A Virus, Influenza B Virus, Respiratorischer Syncytialvirus, Parainfluenza Virus, Rhinovirus und Adenovirus.

4. Konjugat nach einem der Ansprüche 1 - 3, wobei die Viren bindende Substanz ausgewählt ist aus der Gruppe bestehend aus: Virus spezifische Antikörper, Neuraminsäure und deren Derivate, Inhibitoren der Influenza Neuraminidase.

5. Konjugat nach einem der Ansprüche 1 - 4, wobei die Viren bindende Substanz mit dem Polymer kovalent verbunden ist.

6. Mittel geeignet zum Anreichern eines Virus im Mund, Rachen oder in der Nase enthaltend ein Konjugat nach einem der Ansprüche 1 - 5.

7. Mittel nach Anspruch 6 in Form eines Lutschers oder Kaugummis.

8. Komplex bestehend aus einem Konjugat nach einem der Ansprüche 1 - 5 und dem anzureichernden Virus.

9. Verwendung eines Konjugats nach einem der Ansprüche 1 - 5 zur Herstellung eines Diagnostikums zum Nachweis von Viren in Mund, Rachen oder Nase.

10. Verfahren zum Nachweis von Viren, umfassend die Schritte:
a. in vivo Anreicherung der Virus durch ein Konjugat nach einem der Ansprüche 1 - 5
b. Nachweis des Virus in vitro
i) durch enzymatische Aktivität des gebundenen Virus oder
ii) nach Desorption des Virus mit anschließendem oder gleichzeitigem separaten Nachweistest für den Virus.
